# EUROPEAN PATENT SPECIFICATION

(11) **EP 2 490 683 B1**
(45) Date of publication and mention of the grant of the patent: **07.10.2015**
(21) Application number: 10825695.9
(22) Date of filing: 21.10.2010
(51) Int. Cl.: A61K 31/196, A61K 9/00, A61K 9/107, A61K 47/10, A61K 47/14, A61K 47/26, A61K 47/34, A61P 31/22

(54) **DICLOFENAC FOR TREATING HERPES VIRUS INFECTIONS**
DICLOFENAC ZUR BEHANDLUNG VON HERPESVIRUSINFEKTIONEN
DICLOFÉNAC POUR TRAITER LES INFECTIONS PAR LE VIRUS DE L'HERPÈS

(30) Priority: 21.10.2009 US 253813 P
(43) Date of publication of application: 29.08.2012
(73) Proprietor: Yung Shin Pharm. Ind. Co., Ltd., Tachia T'ai chung (TW); Carlsbad Technology Inc, Carlsbad, CA 92008 (US)
(72) Inventor: LEE, FangChen, Taichung Taiwan (TW); SHIEH, Hui-Ling, Taichung Taiwan (TW)
(74) Representative: Schüssler, Andrea
(86) International application number: PCT/US2010/053602
(87) International publication number: WO 2011/050196

(56) References cited:
- EP-A2- 1 457 202
- CN-A- 101 269 058
- US-A- 4 575 515
- US-A1- 2004 068 007
- US-A1- 2009 062 315
- TROUSDALE M D ET AL: "ASSESSMENT OF DICLOFENAC ON HERPES KERATITIS IN RABBIT EYES", ARCHIVES OF OPHTHALMOLOGY, AMERICAN MEDICAL ASSOCIATION, US, vol. 107, no. 11, 1 November 1989 (1989-11-01), pages 1664-1666, XP009033399, ISSN: 0003-9950
- HENDRICKS R L ET AL: "THE EFFECT OF FLURBIPROFEN ON HERPES SIMPLEX VIRUS TYPE 1 STROMAL KERATITIS IN MICE", INVESTIGATIVE OPHTHALMOLOGY AND VISUAL SCIENCE, vol. 31, no. 8, 1990, pages 1503-1511, ISSN: 0146-0404

## Description

### TECHNICAL FIELD

This invention relates to a pharmaceutical composition for use in a method of inhibiting herpes viral activity in a subject by administering to the subject in need thereof an active ingredient consisting essentially of an effective amount of diclofenac, or a pharmaceutically acceptable salt thereof.

### BACKGROUND OF THE INVENTION

The herpes viruses comprise a large family of double stranded DNA viruses. The herpes virus family can be divided into three subfamilies (i.e., a, β, and y) based upon a number of biological properties such as host range and tropism, viral life cycle, and viral persistence and latency. Eight of the herpes viruses, herpes simplex virus types 1 and 2 (HSV-1 and HSV-2), varicella zoster virus (VZV), human cytomegalovirus (HCMV), Epstein-Barr virus (EBV), and human herpes viruses 6, 7, and 8 (HHV-6, HHV-7, and HHV-8), have been shown to infect humans.

Among the herpes viruses, the two commonly known viruses are herpes simplex virus types 1 and 2, referred to as HSV1 and HSV2 and varicella-zoster virus (VZV). HSV1 causes orofacial lesions, commonly known as fever blisters or cold sores. These lesions most commonly appear on the lips, but may appear on the face, in the mucous membrane lining of the oral cavity, in the eye and nose, and occasionally on the trunk of hands. Infections of the mouth are designated with the term herpes labialis, also called cold sore (fever blister). Other parts of the face can also be affected and the infections thereof are referred to as facial herpes simplex. The infection can also manifest itself on other parts of the body. Approximately 30% of the United States population suffers from recurrent episodes of HSV1. HSV2, which is less common than HSV1, causes genital lesions. Conversely, genital herpes is caused in about 30% of cases by HSV1.

Varicella-zoster virus (VZV) causes varicella, commonly known as chicken pox, and herpes zoster, commonly known as shingles. Shingles affects the skin and nerves and is characterized by groups of small blisters or lesions appearing along certain nerve segments. The lesions are most often seen on the back and may be preceded by a dull ache in the affected site.

Once an individual has been infected with the herpes virus, the virus will thereafter remain latently in the body. In latent state, the virus is situated in nerve cell bodies in the ganglia. Due to particular stimuli, such as influenza infection, other respiratory disorders, gastrointestinal infections, stress, fatigue, menstruation, pregnancy, allergy, sunlight, or fever, the latent virus can be activated and travel from the ganglia along the well-defined nerve paths to the skin surface and there multiply and cause the symptoms.

Acyclovir, chemical name acycloguanosine, is a guanosine analogue antiviral drug, targeting viral encoded DNA polymerase. Acyclovir is primarily used for the treatment of herpes simplex virus infections, as well as in the treatment of herpes zoster (shingles). Acyclovir is used to decrease pain and speed the healing of sores or blisters in people who have varicella (chickenpox), herpes zoster, and first-time or repeat outbreaks of genital herpes. Acyclovir is in a class of antiviral medications called synthetic nucleoside analogues. It works by stopping the spread of the herpes virus in the body. Acyclovir will not cure genital herpes and may not stop the spread of genital herpes to other people. Although acyclovir are poorly soluble in water and demonstrate low bioavailability. These, accompanying the relative long recovery time required (i.e., generally takes longer than 2 weeks for patients to recover) and high prescription cost, make the drug less attractive to the patients.

Lidocaine, 2-(diethylamino)-N-(2,6-dimethylphenyl)-acetamide, a local anesthetic, is known for its treatment of ventricular tachycardia (an arrhythmia of the heart) as an intravenous injection solution. Lidocaine is also widely used as a vasoconstrictor to reduce regional blood flow in topical applications or aerosols (such as nasal aerosols to reduce nasal congestion). In addition, lidocaine is known for its therapeutic effects in reducing post-herpetic neuralgia (PHN) nerve injury pain from shingles (herpes zoster and post herpetic neuralgia) and analogous neuropathies.

Lidocaine base is freely lipid soluble. It is insoluble in water and thus not suitable for use in an aqueous suspension, requiring ethanol or the like to obtain a liquid solution. However, its salt form, lidocaine-HCl, is very soluble in water and alcohol. Thus, lidocaine-HCl is generally the form that is used for preparation of injection solution.

Diclofenac, which is 2-(2,6-dichloro-anilino)-phenyl-acetic acid, is known for its role as an anti-rheumatic agent for treatment of rheumatoid arthritis. Diclofenac belongs to the acetic acid class of NSAID (non-steroidal anti-inflammatory drugs). Diclofenac is taken to reduce inflammation and as an analgesic reducing pain in conditions such as arthritis or acute injury. Due to its relatively low solubility in water, an aqueous injection solution of diclofenac is difficult to achieve.

EP1457202 discloses a topical formulation containing a NSAID diclofenac as a sole active agent. The preferred amount of diclofenac used in the topical formulation is about 0.1 -10% by weight (w/w).The topical formulation is used for the treatment of pain/inflammation and skin blisters/lesions caused by herpes virus infection, i.e. herpes simplex virus (HSV) or varicella zoster virus (VZV) infection.

HENDRICKS R L ET AL: "THE EFFECT OF FLURBIPROFEN ON HERPES SIMPLEX VIRUS TYPE 1 STROMAL KERATITIS IN MICE", INVESTIGATIVE OPHTHALMOLOGY AND VISUAL SCIENCE, vol. 31, no. 8, 1990, pages 1503-1511 discloses a NSAID compound flurbiprofen which inhibits HSV-1 replication in Vero cells in a dose-dependent manner. In mice (in vivo) flurbiprofen does not exacerbate HSV-1 stromal keratitis but has an anti-inflammatory efficacy in the treatment of this disease.

US2009062315 discloses a combination of 5 wt % diclofenac acid and lidocaine salt which reduces HSV-1 viral titer.

There exists a need for an effective composition for use in treating herpes virus infection and inhibiting viral activity. The composition does not use multiple drugs and has minimal side effects.

### SUMMARY OF THE INVENTION

A pharmaceutical composition for use in a method of inhibiting herpes viral activity in a subject, wherein the active ingredient of the pharmaceutical composition consists of an effective amount of 5% (w/w) diclofenac or a pharmaceutically acceptable salt thereof, and does not contain any other additional active compound.

The present composition is suitable to inhibit the activity of herpes viruses, including herpes simplex virus type 1 (HSV-1), herpes simplex virus type-2, varicella-zoster virus, and a combination thereof.

### BRIEF DESCRIPTION OF THE DRAWING

Figure 1 shows the inhibition effects on lesion number, lesion area and viral titers by (a) 5% lidofenac and 5% acyclovir, (b) 5% acyclovir, and (c) 5% lidofenac.
Figure 2 compares the inhibition effects on viral titer by lidofenac (1%, 3%, and 5%), diclofenac (5%), lidocaine (5%), and acyclovir (5%).

### DETAILED DESCRIPTION OF THE INVENTION

The inventors have discovered an effective composition for inhibiting herpes viral activity. The inventors have discovered that diclofenac or its pharmaceutically acceptable salt alone as an active compound has an anti-herpes virus effect and is sufficient in reducing herpes viral titers when tested in animals. No additional active compound such as acyclovir or lidocaine is needed to inhibit the herpes virus activity. By using a single drug, the present composition minimizes side effects caused by multiple drugs, improves patient's compliance, and provides an economic advantage.

The present disclosure is directed to a method of inhibiting herpes viral activity in a subject. The method comprises: identifying a subject suffering from herpes virus infection, and topically administering to the subject an active ingredient consisting essentially of an effective amount of diclofenac, or a pharmaceutically acceptable salt thereof, whereby the viral activity is inhibited.

"Pharmaceutically acceptable salts," as used herein, are salts that retain the desired biological activity of the parent compound and do not impart undesired toxicological effects. The pharmaceutically acceptable salts can be formed with metal or organic counterions and include, but are not limited to, alkali metal salts such as sodium or potassium; alkaline earth metal salts such as magnesium or calcium; and ammonium or tetraalkyl ammonium salts, i.e., NX₄+ (wherein X is C₁₋₄). The "pharmaceutically acceptable salts," as used in the application, does not include another active compound as the salt form:
"An effective amount," as used herein, refers to an amount that is effective to reduce and inhibit viral activities. The viral activities can be measured by the virus titer, lesion number, and /or lesion area. An effective amount of diclofenac is about 1-10%, preferably 2-8%, or 3-7%, or 4-6%.

The present composition inhibits the activity of herpes viruses, including herpes simplex virus type 1 (HSV-1), herpes simplex virus type-2, varicella-zoster virus, and a combination thereof.

The present composition topically administers an effective amount of diclofenac or its salt to the afflicted area of a subject. Preferred diclofenac is diclofenac sodium or diclofenac potassium, with diclofenac sodium being more preferred. An effective amount of diclofenac is 1-10%, preferably 2-8%, 3-8%, 3-7%, or 4-6% (w/w). For example, an effective amount of diclofenac is 5% (w/w). The afflicted areas are typically the external tissues of the subject. After the diclofenac treatment, the total lesion number, the lesion size, and the viral titer are reduced.

"About" as used in this application, refers to ± 10% of the recited value. The present invention also provides pharmaceutical compositions comprising one or more pharmaceutically acceptable carrier and the active compound diclofenac, or a pharmaceutically acceptable salt thereof. The active compound, or its pharmaceutically acceptable salt in the pharmaceutical composition in general is in an amount of 1-10%, or 2-8%, or 3-8%, or 3-7%, or 4-6% (w/w). For example, the active compound in the pharmaceutical composition is 1, 3, or 5%.

The pharmaceutically acceptable carrier can be selected by those skilled in the art using conventional criteria. Pharmaceutically acceptable carriers include non-aqueous based solutions, suspensions, emulsions, microemulsions, micellar solutions, gels, and ointments. The pharmaceutically acceptable carriers may also contain ingredients that include saline and aqueous electrolyte solutions; ionic and nonionic osmotic agents such as sodium chloride, potassium chloride, glycerol, and dextrose; pH adjusters and buffers such as salts of hydroxide, hydronium, phosphate, citrate, acetate, and borate; antioxidants such as salts, acids and/or bases of bisulfite, sulfite, metabisulfite, thiosulfite, ascorbic acid, acetyl cysteine, cystein, glutathione, butylated hydroxyanisole, butylated hydroxytoluene, tocopherols, and ascorbyl palmitate; surfactants such as lecithin, phospholipids, including phosphatidylcholine, phosphatidylethanolamine and phosphatidyl inositiol; poloxamers and ploxamines, polysorbates such as polysorbate 80, polysorbate 60, and polysorbate 20, polyethers such as polyethylene glycols and polypropylene glycols; polyvinyls such as polyvinyl alcohol and povidone; cellulose derivatives such as methylcellulose, hydroxypropyl cellulose, hydroxyethyl cellulose, carboxymethyl cellulose and hydroxypropyl methylcellulose and their salts; petroleum derivatives such as mineral oil and white petrolatum; fats such as lanolin, peanut oil, palm oil, soybean oil; mono-, di-, and triglycerides; polymers of acrylic acid such as carboxypolymethylene gel, and polysaccharides such as dextrans, and glycosaminoglycans such as sodium hyaluronate. Such pharmaceutically acceptable carriers may be preserved against bacterial contamination using well-known preservatives, these include benzalkonium chloride, ethylene diamine tetra-acetic acid and its salts, benzethonium chloride, chlorhexidine, chlorobutanol, methylparaben, thimerosal, and phenylethyl alcohol, or may be formulated as a non-preserved formulation for either single or multiple use.

Topical formulations including the active compound can be in a form of gel, cream, lotion, liquid, emulsion, ointment, spray, solution, and suspension.

In one embodiment, the active compound is incorporated into any acceptable carrier, including liquid, creams, gels, lotions or other types of suspensions that can be delivered to the affected area by topical applications. In another embodiment, the pharmaceutical composition can be in the dosage forms such as tablets, capsules, granules, fine granules, powders, syrups, suppositories, injections. The above pharmaceutical composition can be prepared by conventional methods.

The pharmaceutical composition of the present invention can be applied by any of the accepted modes of systemic administration including topical, oral, parenteral (such as intravenous, intramuscular, subcutaneous or rectal), and otherwise systemic routes of administration. The active compound first reaches plasma and then distributes into the target tissues. Dosing of the composition can vary based on the extent of the infection and each patient's individual response. Topical administration is a preferred route of administration for the present invention.

In a preferred embodiment, the composition is applied topically onto the affected area and rubbed into it. The composition is topically applied at least one or two times a day, or 3 to 5 times per day. In general, the topical composition comprises 1-10%, preferably 2-6%, 2-10%, 3-7%, 3-8%, 3-10 %, 4-6%, 4-7%, or 4-8% (w/w) of the active compound. For example, the topical composition comprises 3% or 5% (w/w) of the active compound. Depending on the affected area, typically 0.01-10g, preferably 0.05-5g of the topical composition is applied to the individual per dose. In general, the active compound is applied topically to an individual at 1-500 mg, and preferably 2.5-250 mg/dose. For example, 0.05 g of 5% of dichlofenac sodium (2.5mg) is applied to per cm² of a lesion area. The active compound passes through the skin and is delivered to the site of discomfort.

Those of skill in the art will recognize that a wide variety of delivery mechanisms are also suitable for the present invention.

### EXAMPLES

### Example 1. Preparation of Cream Formulation

A cream vehicle ± [active ingredient] was prepared by the following step:
1. **Preparation of the oil phase:** A mixing vessel was submerged in a hot water bath (80 ± 2 °C). [Acyclovir, 50g] methyl paraben (1g), propyl paraben (1g), cetyl alcohol (60g), sorbitan monostearate 60 (12g), steric acid (20g), spermaceti synthetic (50g), dimethyl polysiloxane (30g), and miglyol 812 (70g) were added into the mixing vessel and stirred to mix well. The mixture was filtered by a number 150 mesh one time to remove particles.
2. **Preparation of the water phase:** Another mixing vessel was submerged in a hot water bath (80 ± 2 °C). [Diclofenac sodium salt, 50g] [Diclofenac acid lidocaine salt, 10g, 30g, or 50g] polysorbate 60 (36g), propylene glycol (160g), sodium citrate (10g), and sufficient purified water to make a total weight 1000g, were added into the mixing vessel and stirred to complete dissolve. The mixture was filtered by a number 150 mesh one time to remove particles.
3. **Emulsification:** The oil phase was transferred into a steam-jacketed tank having a vacuum pressure of 35 - 40 mmHg. The water phase was added slowly in to the steam-jacketed tank at a constant stirring speed by using a Homo-Mixer (25 - 35 Hz) for 25 minutes.
4. The emulsion of the previous step was cooled to a temperature of 30 °C under a slowly stirred condition to obtain a topical formulation in a dosage form of a cream.

### Example 2. Test Different Compounds on Animals

### Objectives:

Effects of (1) ADO-1, ADO-2, ADO-3, and ADO-4 ; (2) 1% VDO99 Cream, 3% VDO99 Cream, 5% VDO99 Cream and their placebos; (3) VGO99 Cream (Diclofenac Sodium, 50mg (RA009)), VDO99 Cream (Diclofenac Acid, Lidocaine Salt 50mg (RA052)), VAO99 Cream (Lidocaine, 50mg (RA001)) and their placebo creams were tested on Herpes Animal Model. The cream formation ± active ingredient was prepared according to Example 1.

### Compounds Tested:

### Group I

ADO-1: 5% Acyclovir (50mg/g) plus 5% Diclofenac Acid Lidocaine Salt (Lidofenac 50mg/g)
ADO-2: 5% Acyclovir (50mg/g)
ADO-3: 5% Diclofenac Acid Lidocaine Salt (Lidofenac 50mg/g)
ADO-4: Vehicle.

### Group II

1% VDO99 Cream: Diclofenac Acid, Lidocaine salt (Lidofenac 10mg/g, RA032); VDO99 Placebo Cream (RA035 Placebo);
3% VDO99 Cream: Diclofenac Acid, Lidocaine salt (Lidofenac 30mg/g, RA033); VDO99 Placebo Cream (RA036 Placebo);
5% VDO99 Cream: Diclofenac Acid, Lidocaine salt (Lidofenac 50mg/g, RA034); VDO99 Placebo Cream (RA037 Placebo).

### Group III

1% VDO99 Cream: Diclofenac Acid, Lidocaine salt (Lidofenac 10mg/g, RA032); VDO99 Placebo Cream (RA035 Placebo);
3% VDO99 Cream: Diclofenac Acid, Lidocaine salt (Lidofenac 30mg/g, RA033); VDO99 Placebo Cream (RA036 Placebo);
5% VGO 99 Cream, Diclofenac Sodium, 50mg/g (RA009), Placebo Cream (RA010);
5% VDO 99 Cream, Diclofenac Acid, Lidocaine Salt (Lidofenac 50mg/g, RA052), Placebo Cream (RA053);
5% VAO99 Cream, Lidocaine, 50mg/g (RA001), Placebo Cream (RA003).

The compounds were kept at room temperature in the dark when not in use. 5% ACV/PEG (Zovirax Ointment) was used as a control in the experiment.

### Animal Inoculation

Hartley outbred albino guinea pigs were used in this experiment. HSV-1 virus stock (0.035 ml) was applied to each area and introduced through the skin at well-spaced sites by ten activations of a six-pronged spring-loaded vaccination instrument (Sterneedle, Pan Ray Division, Ormont Drug, Englewood, New Jersey). The day of inoculation was Day 0. Approximately 250 mg of test drugs were applied at 4x/day on Days 1, 2 and 3.

The dosing regimen for 5% ACV/PEG (Zovirax Ointment) was reduced from our standard dosing regimen of 5x/day for Days 1, 2 and 3 to 4x/day for Days 1, 2 and 3 to match the regimen of the Group I.

For Group II and Group III, the dosing regimen for 5% ACV/PEG (Zovirax Ointment) of our standard dosing regimen of 5x/day for Days 1, 2 and 3 were used as experimental control.

On Day 4, the sites on the backs of the animals were treated with Nair once to remove regrown hair on the animals' backs. Lesions were counted from the animals and pictures of the animals were taken and used later to measure lesion sizes. The animals were sacrificed and full-thickness skin from the different areas was removed. The rectangle of skin from each of the treatment areas was placed into 15 mls of tissue culture medium with 2% fetal bovine serum (FBS) in an ice bath. The samples were then homogenized in a Stomacher 80 Biomaster lab blender (Seward Co.). Debris was pelleted by centrifugation and the supernatants were collected and frozen at -70°C until assay for infectivity by plaque formation in VERO 76 cells (Kidney, African green monkey, ATCC CRL# 1587).

### Dermal Irritation

We tested (1) ADO-1, ADO-2, ADO-3, and ADO-4 ; (2) 1% VDO99 Cream, 3% VDO99 Cream, 5% VDO99 Cream and their placebos; (3) VGO99 Cream (Diclofenac Sodium, 50mg (RA009)), VDO99 Cream (Diclofenac Acid, Lidocaine Salt 50mg (RA052)), VAO99 Cream (Lidocaine, 50mg (RA001)) and their placebo creams for dermal irritation using dosing regimens of 1x/day, 2x/day, 4x/day and 5x/day, all on Days 1, 2 and 3 on uninfected Hartley outbred albino guinea pigs.

### Group I: ADO-1, ADO-2, ADO-3, and ADO-4

The 5x/day dosing regimen was stopped early because there appeared to be a shortage of compounds ADO-1, -2 and -3 for testing in infected animals.

All the compounds, including the vehicle, were moderately irritating at both the 2x/day and 4x/day dosing regimens. But it was decided to use the 4x/day dosing regimen.

### Group II: 1% VDO99 Cream, 3% VDO99 Cream, 5% VDO99 Cream and their placebos

All the compounds, including the placebos, were moderately to severely irritating at both the three dosing regimens. In conjunction with the sponsor, we decided to use the most potent compound, 5% VDO99, and its placebo at the 2x/day dosing regimen. Even at that dosing regimen we understood the dermal irritation caused by the 5% VDO99 and its vehicle might prevent us from visualizing lesions on Day 4 to count and measure them. This turned out to be the case.

### Group III: VGO99 Cream (Diclofenac Sodium, 5% (RA009)), VDO99 Cream (Diclofenac Acid, Lidocaine Salt 50mg (RA052)), VAO99 Cream (Lidocaine, 50mg (RA001)) and their placebo creams

VGO 99 Cream and VDO99 Cream, including their placebos, were moderately to severely irritating at all three dosing regimens. VAO 99 Cream was mildly irritating at 1x and 2x/day and moderately to severely irritating at 4x/day on Days 1, 2 and 3.

1% VDO99 Cream, 3% VDO99 Cream, and their placebo creams were moderately to severely irritating at all three dosing regimens. We decided to use at the 2x/day dosing regimen. Even at that dosing regimen we understood the dermal irritation caused by the compounds and their vehicles likely would prevent us from visualizing lesions on Day 4 to count and measure them. This turned out to be the case. On Day 4, with infected animals, dermal irritation prevented us for efficacy evaluation:
3 of 12 drug/placebo cream pairs for 1% VD099;
6 of 12 drug/placebo cream pairs for 3% VD099;
11 of 12 drug/placebo cream pairs for VG0 99;
8 of 12 drug/placebo cream pairs for VD0 99;
7 of 12 drug/placebo cream pairs for VA0 99.

### Results

### Group I

Ten active vs. vehicle pairs were tested.
The infection with HSV-1 exacerbated the irritation caused by the active compounds, including the vehicle, and made it very difficult to see the lesions to count them on Day 4. This sometimes happens in this model and is difficult to predict. The irritation also made it very difficult to measure the lesions from the pictures taken on Day 4. Because of these difficulties the number of comparisons for Lesion Number and Total Lesion Area were reduced and lessened the chance that "n" would be large enough to achieve significance.

Compounds that are well-tolerated in humans can often be irritating to the animals in our model so that dermal irritation in our model may not be a predictor of dermal irritation in humans.

Table 1 shows the results of analysis (InStat. V3, GraphPad Software, Inc.), with the level of significance of p ≤ 0.05 for the experiments.

When compared to the vehicle (ADO-4), ADO-1 achieved statistically significant reductions in Lesion number, 26%, and 47% in Total Lesion Area. It showed a trend in reducing Virus Titer.

When compared to the vehicle (ADO-4), ADO-2 achieved statistically significant reductions of 26% in Total Lesion Area and 51 % in Virus Titer. It showed a trend in reducing Lesion Number.

When compared to the vehicle (ADO-4), ADO-3 only achieved a statistically significant reduction of 86% in Virus Titer. Since ADO-3 appeared to be the most irritating in infected animals the results, or lack of them, for Lesion Number and Total Lesion Area need to be understood in that context.

5% ACV/PEG, when compared to its vehicle, achieved significant reduction in Total Lesion Area. These results for 5% ACV/PEG are not typical for our model but we generally use a dosing regimen of 5x/day on Days 1,2 and 3. We typically see significant decreases in Total Lesion Area and Virus Titer, results that would be very similar to ADO-2.

### Group II

1% VD099, 3% VD099, 5% VD099 and their respective vehicles were all moderately to severely irritating to the animals' skin. In conjunction with the sponsor and in an effort to preserve resources, we decided to use the most potent compound, 5% VD099 and its placebo, at a dosing regimen of 2x/day on Days 1, 2 and 3.

The infection with HSV-1 exacerbated the irritation caused by the tested compounds, including the vehicle, and made it very difficult to see the lesions to count them on Day 4. This sometimes happens in this model. The irritation also made it very difficult to measure the lesions from the pictures taken on Day 4. Because of these difficulties the number of comparisons for Lesion Number and Total Lesion Area were reduced and lessened the chance that "n" would be large enough to achieve significance.

Table II shows the results of analysis (InStat. V3, GraphPad Software, Inc.), with the level of significance of p ≤ 0.05 for the experiments.

When compared to its placebo, 5% VD099 achieved a statistically significant reduction of 23% in Lesion number, a statistically significant reduction of 26% in Total Lesion AREA, and a statistically significant reduction of 52% in Virus Titer.

5% ACV/PEG, when compared to its vehicle; achieved significant reductions in Lesion Number, 10%; Total Lesion Area, 33% and Virus Titer, 79%. These results for 5% ACV/PEG are typical for our model. They also reflect the general pattern of efficacy we see for true antiviral medications in our model: the reduction in Virus Titer is greater than the reduction in Total Lesion Area which is greater than the reduction in Lesion Number. (Virus Titer reduction > Total Lesion Area reduction > Lesion Number reduction.)

### Group III

1% VD099, 3% VD099, VG0 99, VD0 99, VA0 99 and their respective vehicles were all moderately to severely irritating to the animals' skin.

The infection with HSV-1 exacerbated the irritation caused by the tested compounds, including their vehicles, and made it very difficult to see the lesions to count them on Day 4. This sometimes happens in this model. The irritation also made it very difficult to measure the lesions from the pictures taken on Day 4. Because of these difficulties the number of comparisons for Lesion Number and Total Lesion Area were reduced and lessened the chance that "n" would be large enough to achieve significance, or even do the statistical test.

Table III shows the results of analysis (InStat. V3, GraphPad Software, Inc.), with the level of significance of p ≤ 0.05 for the experiments.

The most notable result is that, when compared to its placebo cream, VGO 99 achieved a statistically significant reduction of 93% in Virus Titer.

5% ACV/PEG, when compared to its vehicle, achieved significant reductions in Total Lesion Area, 27% and Virus Titer, 71%. These results for 5% ACV/PEG are typical for our model. They also reflect the general pattern of efficacy we see for true antiviral medications in our model: the reduction in Virus Titer is greater than the reduction in Total Lesion Area which is greater than the reduction in Lesion Number. (Virus Titer reduction > Total Lesion Area reduction > Lesion Number reduction.)

### Summary of Results

### Group I

ADO-3, 5% lidofenac achieved 86% reduction in Virus Titer.
ADO-1, the combination of acyclovir and lidofenac, did not achieve the reduction in virus titer as good as lidofenac. However, ADO-1 achieved smaller but significant reductions in Lesion Number and Total Lesion Area.

### Group II

5% VDO99 (RA034); 5% lidofenac, achieved modest but significant results in all three measures of efficacy in our model. Both 5% VDO99 and its vehicle were also equally irritating to the animals' skin.

### Group III

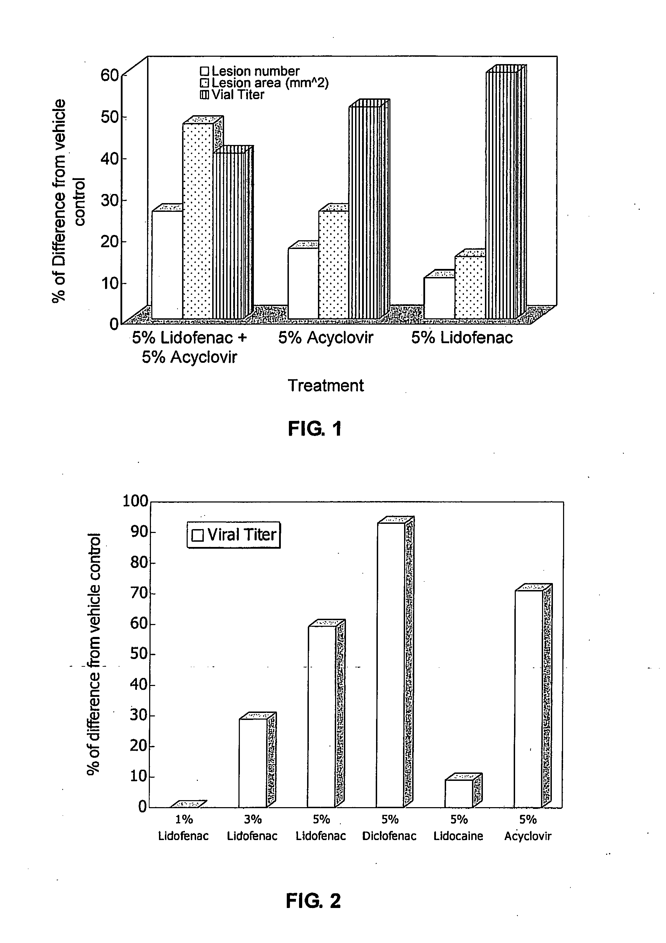

VGO99 (RA009), diclofenac sodium, 50mg/g (5% w/w), achieved a 93% reduction in virus titer in our model. Both VG0 99 and its placebo cream were also equally irritating to the animals' skin, preventing us from evaluating the effect the compound might have had on reducing lesion number and total lesion area. The Results are also summarized in Figures 1 and 2. Figure 1 shows the inhibition effects in lesion number, lesion area and viral titers by (a) 5% lidofenac and 5% acyclovir, (b) 5% acyclovir, and (c) 5% lidofenac. Since there were three data points of 5% lidofenac, the data for 5% lidofenac in the graph represent the average of the 3 sets of data. Figure 1 shows that 5% acyclovir plus 5 % lidofenac had the highest inhibition effect in lesion number and lesion area, and 5 % lidofenac showed highest inhibition effect in viral titer.

Figure 2 compares the inhibition effects of lidofenac (1%, 3%, and 5%), diclofenac (5%), lidocaine (5%), and acyclovir (5%), on viral titer. Lidofenac (1%, 3%, and 5%) showed a dose response of inhibition effect in viral titer. 5% diclofenac showed a very good inhibition effect in viral titer. 5% lidocaine only had a small inhibition effect on viral titer.

**Table I**

| Efficacy Analysis for ADO-1, -2, -3 vs. Veh (ADO-4); And 5% ACV/PEG vs. PEG. | | | | | | | | | | | | |
|---|---|---|---|---|---|---|---|---|---|---|---|---|
| | ADO-1 | % Diff (p) | Veh ADO-4 | ADO-2 | % Diff (p) | Veh ADO-4 | ADO-3 | % Diff (p) | Veh ADO-4 | 5% ACV/ PEG | % Diff (p) | PEG. |
| Lesion Number² | | | | | | | | | | | | |
| mean | 39 | 26¹ | 53 | 44 | 17 | 53 | 44 | 10 | 49 | 52 | -- | 52 |
| sd | 11 | (.003)³ | 4 | 13 | (.12) | 4 | 14 | (.12) | 6 | 5 | (.94) | 7 |
| n | 8 | | 8 | 6 | | 8 | 6 | | 9 | 10 | | 10 |
| median | 39.0 | | 51.5 | 47.0 | | 52.5 | 48.5 | | 50.0 | 51.0 | | 53.0 |
| Total Lesion Area mm2 | | | | | | | | | | | | |
| mean | 118 | 47 | 222 | 171 | 26 | 232 | 184 | 15 | 217 | 159 | 18 | 207 |
| sd | 26 | (.0003) | 35 | 26 | (.049) | 25 | 46 | (.056) | 26 | 29 | (.002) | 39 |
| n | 8 | | 8 | 6 | | 8 | 5 | | 8 | 10 | | 10 |
| median | 126.5 | | 220.0 | 178.0 | | 233.0 | 180.0 | | 207.5 | 161.0 | | 212.5 |
| Virus Titer log (pfu/ml) | | | | | | | | | | | | |
| mean | 3.72 | 40 | 3.94 | 3.69 | 51 | 4.00 | 3.23 | 86 | 4.09 | 3.80 | 24 | 3.92 |
| sd | .33 | (.12) | .30 | .60 | (.043) | .44 | .53 | (.015) | .50 | .45 | (.23) | .48 |
| n | 10 | | 10 | 10 | | 10 | 10 | | 12 | 10 | | 10 |
| median | 3.80 | | 3.94 | 3.88 | | 3.97 | 3.39 | | 4.11 | 3.78 | | 3.98 |

| | | | | | | | | | | | | |
|---|---|---|---|---|---|---|---|---|---|---|---|---|
| ADO-1: Acyclovir plus Diclofenac Acid Lidocaine Salt. ADO-2: Acyclovir ADO-3: Diclofenac Acid Lidocaine salt. ADO-4: Vehicle. 1. Percent differences between mean lesion severity at drug-treated sites compared to the vehicle-treated sites are shown. A positive value indicates a reduction in lesion severity for the test compound. 2. Day 0 is the day of infection. All compounds were used 4x/day on Days 1,2 and 3. Efficacy measurements were done on the morning of Day 4. 3. For statistical analysis, paired data were evaluated by the paired t test, utilizing the means of the drug and vehicle results. | | | | | | | | | | | | |

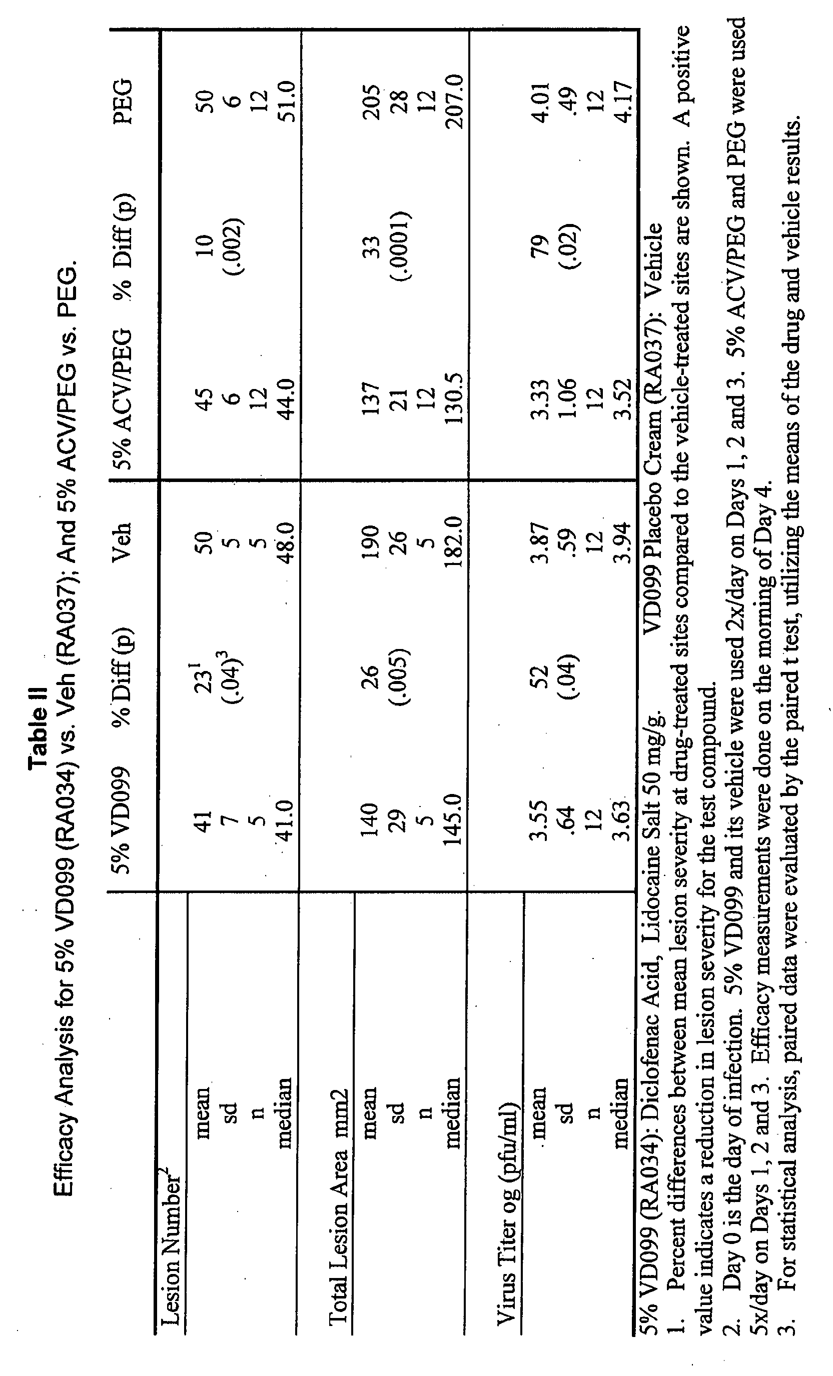

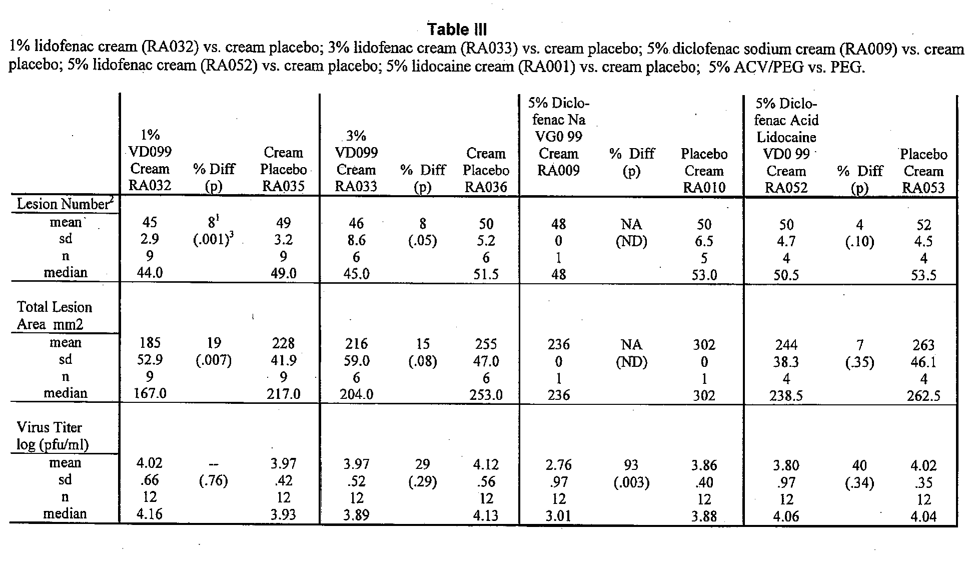

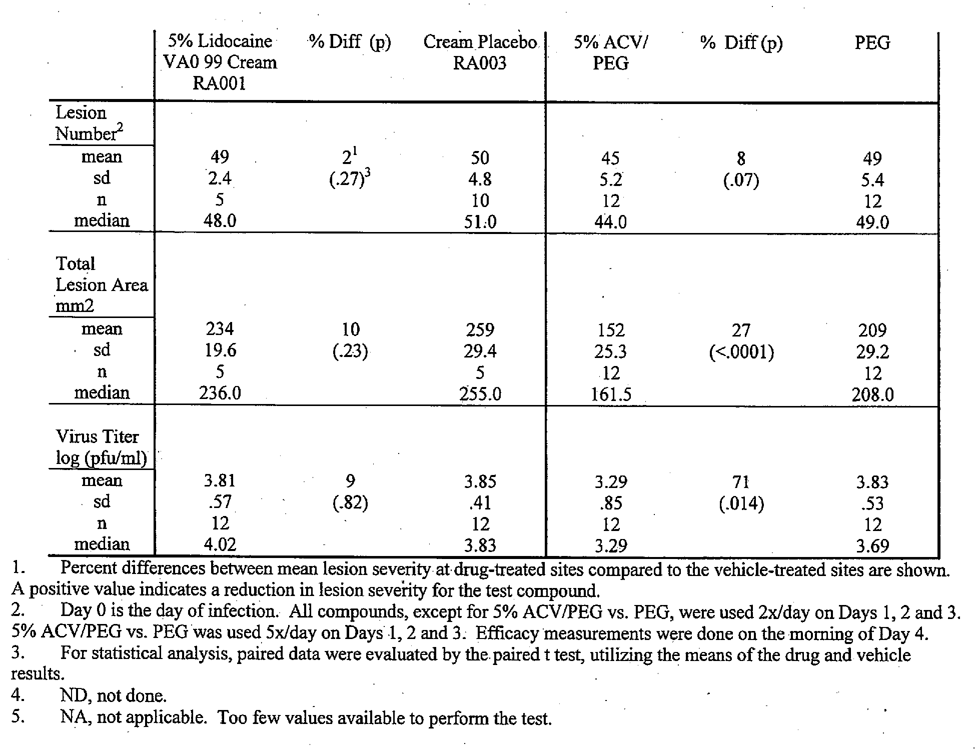

### Example 3. Clinical Study Protocol

### Objectives:

To evaluate the effectiveness of VGO99 (5% diclofenac sodium cream) in patients with herpes zoster. The efficacy is judged by the intensity of pain and lesion assessment.

### Study Endpoint

The following endpoints are evaluated:
- The primary efficacy endpoint is time to complete cessation of zoster-associated pain. Subjects are defined as achieving complete cessation of pain if they are pain-free (pain reported to be 0 on a 0 ~ 100 numerical rating scale) for at least 7 days.
- Time to loss of acute phase pain (pain experienced up to the point when all crusts are lost)
- The crude rate of subjects achieving complete cessation of zoster-associated pain at the end of follow-up period.
- The crude rate of subjects without vesicles, ulcers and crusts at the end of treatment period and at the end of follow-up period.
- The percent reduction in VAS pain score at specific clinic visits after study medication administration.
*Treatment regimens:* Topical VGO99 cream, 10g/tube, topical use on the lesion
*Control regimens:* Topical placebo cream, 10g/tube, topical use on the lesion

### Subjects

- Male or female aged from 20 years to 75 years.
- Subjects with a clinical diagnosis of uncomplicated herpes zoster by localized, cutaneous lesions (papules, macules or vesicles).
- Subjects to whom the study drugs can be administered within 72 hours after the onset of rash and/or bud vesicles due to herpes zoster.
- Subjects at screening/baseline must have a score of at least 40 mm on the visual analog scale (VAS).
- Ability to understand and follow the instructions of the investigator, including completion of the study diaries as described in the protocol.

### Test Protocols

Subjects who meet all the selection criteria are randomly assigned to receive one of the following treatments for 28 days:
Group I: VGO99 cream (5% diclofenac sodium)
Group II: placebo cream
Patients are randomized to receive either VGO99 cream or placebo cream about 0.05g/cm², 4 times daily until complete cessation of zoster-associated pain, but no longer than 28 days. After the last dose of study medication administration, all subjects are followed for 4 weeks.

This study lasts about 8 weeks and includes 7 study visits on Day 0 (baseline), 3±1, 7±1, 14±2, 21±2, 28±2 and 56±7. All study visits include questionnaires on pain levels and an examination of the affected skin area.

### Statistic Methods

For the efficacy endpoints, Kaplan-Meier method is conducted for time to events variable (including time to complete cessation of zoster-associated pain and time to loss of acute phase pain), and the median along with two-sided 95% confidence interval is reported. The log-rank test is used for comparing times to event among two groups of a topical preparation where the subject is treated for 28 days or until the specific event achieved, whichever comes first. The Cox regression model is used to analyze event times among treatment groups with adjustments for age, the time from rash onset to treatment initiation, and/or other significant prognostic index.

## Claims

1. A pharmaceutical composition for use in a method of inhibiting herpes viral activity in a subject, wherein the active ingredient of the pharmaceutical composition consists of an effective amount of 5% (w/w) diclofenac or a pharmaceutically acceptable salt thereof, and does not contain any other additional active compound.

2. The pharmaceutical composition for the use according to claim 1, wherein the herpes virus is selected from the group consisting of herpes simplex virus type 1 (HSV-1), herpes simplex virus type-2, varicella-zoster virus, and a combination thereof.

3. The pharmaceutical composition for the use according to claim 1, which is a topical formulation.

4. The pharmaceutical composition for the use according to claim 3, wherein the topical formulation is in the form of a gel, cream, ointment or lotion.

5. The pharmaceutical composition for the use according to claim 3, wherein the composition is topically applied at least one or two times a day, or 3 to 5 times per day.

## Patentansprüche

1. Pharmazeutische Zusammensetzung zur Verwendung in einem Verfahren zur Hemmung der Aktivität von Herpes-Virus in einem Subjekt, wobei der aktive Wirkstoff der pharmazeutischen Zusammensetzung aus einer wirksamen Menge von 5% (w/w) Diclofenac oder einem pharmazeutisch verträglichen Salz davon besteht, und keine andere aktive Verbindung umfasst.

2. Pharmazeutische Zusammensetzung zur Verwendung nach Anspruch 1, wobei der Herpes-Virus ausgewählt ist aus der Gruppe, bestehend aus Herpes-Simplex Virus Typ 1 (HSV-1), Herpes-Simplex Virus Typ 2, Varizella-Zoster Virus und einer Kombination davon.

3. Pharmazeutische Zusammensetzung zur Verwendung nach Anspruch 1, die eine topische Formulierung ist.

4. Pharmazeutische Zusammensetzung zur Verwendung nach Anspruch 3, wobei die topische Formulierung in Form eines Gels, Creme, Salbe oder Lotion ist.

5. Pharmazeutische Zusammensetzung zur Verwendung nach Anspruch 3, wobei die Zusammensetzung mindestens einmal oder zweimal am Tag, oder 3 bis 5 Mal pro Tag topisch verabreicht wird.

## Revendications

1. Composition pharmaceutique destinée à être utilisée dans un procédé d'inhibition de l'activité virale de l'herpès chez un sujet, dans laquelle l'ingrédient actif de la composition pharmaceutique consiste en une quantité efficace de 5% (p/p) de diclofénac ou un sel pharmaceutiquement acceptable de ce dernier, et ne contient aucun autre composé actif additionnel.

2. Composition pharmaceutique destinée à être utilisée selon la revendication 1, dans laquelle le virus de l'herpès est choisi parmi le groupe composé du virus simplex de l'herpès du type 1 (HSV-1), du virus simplex de l'herpès du type 2, du virus de la varicelle et du zona, et d'une combinaison de ces derniers.

3. Composition pharmaceutique destinée à être utilisée selon la revendication 1, qui est une formulation topique.

4. Composition pharmaceutique destinée à être utilisée selon la revendication 3, dans laquelle la formulation topique est sous forme d'un gel, d'une crème, d'une pommade ou d'une lotion.

5. Composition pharmaceutique destinée à être utilisée selon la revendication 3, dans laquelle la composition est appliquée par voie topique au moins une ou deux fois par jour, ou de 3 à 5 fois par jour.
